(19) Europäisches Patentamt
European Patent Office
Office européen des brevets

(11) Numéro de publication: **0 022 762**
**B1**

# (12) FASCICULE DE BREVET EUROPEEN

(45) Date de publication du fascicule de brevet: **25.11.81**

(21) Numéro de dépôt: **80870035.5**

(22) Date de dépôt: **30.06.80**

(51) Int. Cl.³: **C 07 D 471/04,**
A 61 K 31/44 //(C07D471/04, 221/00, 209/00)

## (54) Nouveaux dérivés d'indolizine, leur procédé de préparation ainsi que leurs applications en thérapeutique.

(30) Priorité: **06.07.79 GB 7923599**

(43) Date de publication de la demande:
**21.01.81 Bulletin 81/3.**

(45) Mention de la délivrance du brevet:
**25.11.81 Bulletin 81/47**

(84) Etats Contractants Désignés:
**BE CH DE FR GB IT LI LU NL SE**

(56) Documents cités:
**GB - A - 1 518 443**

(73) Titulaire: **S.A. LABAZ N.V.**
**Avenue De Béjar, 1**
**B-1120 Bruxelles (BE)**

(72) Inventeur: **Rosseels, Gilbert**
**Uilenspiegelpark 39**
**B-1811 Relegem (BE)**
Inventeur: **Nokin, Pierre**
**Avenue des Bécassines 29**
**B-1160 Bruxelles (BE)**

(74) Mandataire: **Cauchie, Daniel et al,**
**c/o S.A. LABAZ N.V. Avenue De Béjar, 1**
**B-1120 Bruxelles (BE)**

**EP 0 022 762 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Nouveaux dérivés d'indolizine, leur procédé de préparation ainsi que leurs applications en thérapeutique

La présente invention se rapporte, d'une manière générale, à des composés hétérocycliques et en particulier à de nouveaux dérivés d'indolizine ainsi qu'à leur procédé de préparation.

Les dérivés d'indolizine de l'invention peuvent être représentés par la formule générale:

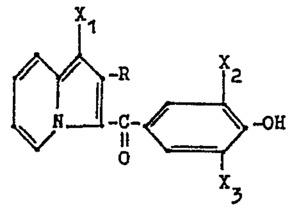

I

dans laquelle R représente un radical alkyle, linéaire ou ramifié, ayant de 1 à 8 atomes de carbone, un groupement phényle ou un groupement phényle portant un ou deux substituants, identiques ou différents choisis parmi les atomes d'halogène, par exemple le fluor, le chlore et le brome et parmi des groupements alkyles inférieurs et alkoxy inférieurs, par exemple, méthyle et méthoxy et $X_1$, $X_2$ et $X_3$, qui sont identiques ou différents, représentent chacun hydrogène chlore, brome ou méthoxy, à condition que lorsqu'ils sont identiques $X_1$, $X_2$ et $X_3$ ne sont pas simultanément hydrogène.

L'invention se rapporte, notamment, aux composés de formule I dans laquelle R représente un radical alkyle, linéaire ou ramifié, ayant de 1 à 8 atomes de carbone, un radical phényle, un radical monofluoro-, monochloro-, monobromo-, monométhyl- ou monométhoxy-phényle, un radical difluoro-, dichloro-, dibromo-phényle ou un radical méthyl-phényle substitué dans la portion aromatique par un atome de fluor, de chlore ou de brome et $X_1$, $X_2$ et $X_3$, qui sont identiques ou différents, représentent chacun hydrogène, chlore, brome ou méthoxy, à condition que lorsqu'ils sont identiques, $X_1$, $X_2$ et $X_3$ ne sont pas simultanément hydrogène.

On a trouvé que les dérivés de l'invention possédent d'utiles propriétés pharmacologiques et plus particulièrement une activité inhibitrice de l'action de la xanthine oxydase et de l'adénosine désaminase ainsi qu'une action uricosurique. En outre, on a trouvé que des composés de l'invention potentialisent les effets cardiovasculaires des catécholamines.

Un autre objet de l'invention se rapporte aux dérivés d'indolizine de formule I utiles pour le traitement de désordres physiologiques consécutifs à un excès d'acide urique et de perturbations du système immunitaire.

Les désordres physiologiques consécutifs à un excès d'acide urique pourront être traités chez l'être humain en administrant par exemple 300 mg/jour de principe actif selon l'invention. A titre d'exemples de tels troubles liés à un excès d'acide urique, on peut citer la goutte et la goutte tophacée.

Quant aux perturbations du système immunitaire qui peuvent être traitées au moyen de composés de l'invention, on peut mentionner, comme exemple, la prévention du phénomène de rejet lors de transplantations d'organes.

Selon un autre aspect de l'invention, celle-ci se rapporte à des dérivés d'indolizine de formule I utiles dans les désordres cardiovasculaires qui peuvent être traités en potentialisant les effets cardiovasculaires des catécholamines par exemple les troubles du système vasculaire cérébral et l'insuffisance cardiaque.

La présente invention concerne, en outre, des compositions pharmaceutiques ou vétérinaires contenant, comme principe actif, au moins un dérivé d'indolizine de formule I en association avec un véhicule pharmaceutique ou un excipient approprié.

L'invention se rapporte également à un procédé de préparation de compositions pharmaceutiques ou vétérinaires, procédé suivant lequel on associe au moins un dérivé d'indolizine de formule I à un véhicule pharmaceutique ou un excipient approprié.

Les composés de formule I peuvent être préparés suivant différents procédés eu égard à leur structure chimique.

Ainsi, les composés de l'invention peuvent être obtenus comme suit:

(A) Lorsque $X_1$ représente hydrogène ou méthoxy, $X_2$ représente chlore, brome ou méthoxy et $X_3$ représente hydrogène, en condensant, dans un solvant approprié tel que le dichloréthane, une alkyl-2 ou aryl-2 indolizine de formule générale:

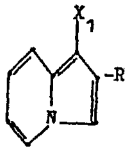

II

dans laquelle $X_1$ représente hydrogène ou méthoxy et R a la même signification que précédemment, avec un dérivé de chlorure d'acide benzoïque de formule générale:

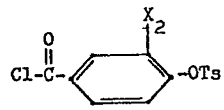

III

dans laquelle $X_2$ représente chlore, brome ou méthoxy et Ts représente un radical p-toluènesulfonyle, pour obtenir un dérivé d'indolizine de formule générale:

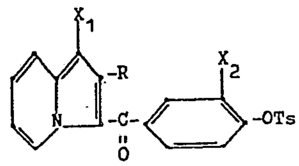

IV

dans laquelle $X_1$ représente hydrogène ou méthoxy, $X_2$ représente chlore, brome ou méthoxy et Ts a la même signification que précédemment, que l'on chauffe au reflux en présence d'un hydroxyde de métal alcalin, par exemple l'hydroxyde de sodium, ce qui fournit le dérivé métallique correspondant que l'on hydrolyse, par la suite à température ambiante et en présence d'un acide hydrohalique, par exemple l'acide chlorhydrique, pour obtenir le composé désiré de formule I.

La condensation sera effectuée à température ambiante ou en chauffant légèrement le milieu réactionnel à environ 50°C.

(B) Lorsque $X_1$ représente hydrogène ou méthoxy et $X_2$ et $X_3$, qui sont différents représentent chacun chlore, brome ou méthoxy, en condensant, à température ambiante et dans un solvant approprié par exemple le dichloréthane, une alkyl-2 ou aryl-2 indolizine de formule générale II, avec un dérivé de chlorure d'acide p-hydroxybenzoïque de formule générale:

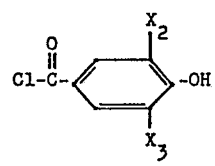

V

dans laquelle $X_2$ et $X_3$, qui sont identiques ou différents, représentent chacun chlore, brome ou méthoxy, ce qui fournit le composé désiré de formule I.

(C) Lorsque $X_1$ représente brome et $X_2$ et $X_3$, qui sont identiques ou différents, représentent chacun hydrogène, chlore, brome ou méthoxy, en faisant réagir le brome avec un dérivé de benzoyl-indolizine de formule générale:

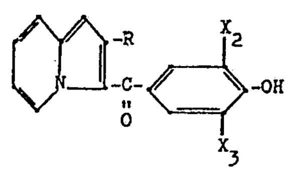

VI

dans laquelle R a la même signification que précédemment et $X_2$ et $X_3$, qui sont identiques ou différents, représentent chacun hydrogène, chlore, brome ou méthoxy, la réaction ayant lieu à la température ambiante dans un solvant approprié tel que le dioxane et en présence d'un acétate de métal alcalin par exemple l'acétate de sodium, pour obtenir le composé désiré de formule I.

L'acide acétique peut également constituer un autre solvant approprié. Cependant, on n'utilisera ce solvant que lorsque le composé de départ de formule VI est monosubstitué par le chlore, le brome ou un radical méthoxy sur l'entité benzoyle.

Le procédé ainsi décrit peut être mis en oeuvre en mettant un équivalent molaire de composé de formule VI en présence de un, deux ou trois équivalents molaires de brome. Cependant, on a observé que:

— lorsqu'un équivalent molaire de brome est utilisé, l'halogénation s'effectue sélectivement en position 1 du cycle indolizine du dérivé benzoyl-indolizine de formule VI, ce composé de formule VI étant non substitué, monosubstitué ou disubstitué par le chlore, le brome ou par un radical méthoxy sur l'entité benzoyle.

— lorsque deux équivalents molaires de brome sont utilisés, l'halogénation se produit non seulement en position 1 du cycle indolizine du composé de formule VI mais également en positions 3 et 5 de l'entité benzoyle. Pour cette raison, le procédé décrit précédemment

sera de préférence entrepris au départ d'un composé de formula VI portant un atome de chlore ou de· brome ou un radical méthoxy sur l'entité benzoyle, de façon à éviter des mélanges de composes halogénés.

Il est évident que lorsqu'un équivalent molaire de (chloro-3 ou bromo-3 ou méthoxy-3 hydroxy-4 benzoyl)-3 indolizine substituée en position 2 est utilisé en presence de deux équivalents molaires de brome, il sera nécessaire de procéder à une séparation du mélange de dérivés halogénés ainsi obtenus. Cette séparation peut être entreprise suivant des méthodes classiques, par exemple par chromatographie sur colonne.

— lorsque trois équivalents molaires de brome sont utilisés en présence d'un équivalent molaire de composé de formule VI à savoir de (hydroxy-4 benzoyl)-3 indolizine substituée en position 2, le dérivé trihalogéné correspondant de formule I est obtenu de manière sélective.

(D) Lorsque $X_1$ représente chlore et $X_2$ et $X_3$, qui sont identiques ou différents, représentent chacun hydrogène, chlore, brome ou méthoxy, en faisant réagir la N-chlorosuccinimide avec un dérivé de benzoyl-indolizine de formule générale:

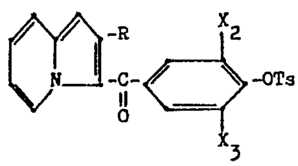

VII

dans laquelle R et Ts ont la même signification que précédemment et $X_2$ et $X_3$, qui sont identiques ou différents, représentent chacun hydrogène, chlore, brome ou méthoxy, la réaction ayant lieu dans un solvant approprié tel que le dichloréthane et entre 0°C et la température ambiante, pour obtenir un dérivé d'indolizine de formule générale:

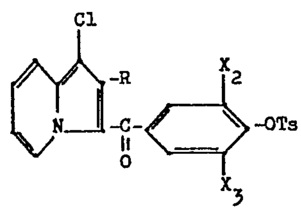

VIII

dans laquelle R, Ts, $X_2$ et $X_3$ ont la signification donnée dans la formule VII, que l'on chauffe au reflux en présence d'un hydroxyde de métal alcalin, par exemple l'hydroxyde de sodium, ce qui fournit le dérivé de métal alcalin correspondant que l'on hydrolyse, par la suite, en présence d'un acide hydrohalique, par exemple l'acide chlorhydrique, ce qui fournit le composé désiré de formule I.

Le procédé ainsi décrit, sera mis en oeuvre en mettant un équivalent molaire de composé de formule VII avec un équivalent molaire de N-chlorosuccinimide, cet imide étant, de préférence utilisé en léger excés.

Lorsqu'on fait réagir un équivalent molaire de composé de formule VII avec un équivalent molaire de N-chlorosuccinimide, l'halogénation s'effectue sélectivement en position 1 du cycle indolizine du dérivé benzoylindolizine de formule VII, ce composé de formule VII étant non substitué, monosubstitué ou disubstitué par le chlore, le brome ou par un radical méthoxy sur l'entité benzoyle.

La structure chimique des composés préparés selon le procédé ci-dessus a été déterminée en examinant le spectre R.M.N. des composés en question.

On a trouvé que cette structure chimique correspond à celle des dérivés d'indolizine représentés par la formule I précédente.

Par exemple, on a prouvé que lorsqu'une (hydroxy-4 benzoyl)-3 indolizine substituée en position 2 est bromée selon le procédé (C) ci-dessus, de façon a obtenir un dérivé monobromé, l'atome de brome se fixe en position 1 du cycle indolizine:

on conserve les caractéristiques d'un noyau aromatique p-disubstitué et on n'observe pas de singulet pour le proton $H_1$.

A titre d'exemple, on a cité ci-dessous les caractéristiques enregistrées avec la bromo-1 éthyl-2 (hydroxy-4 benzoyl)-3 indolizine.

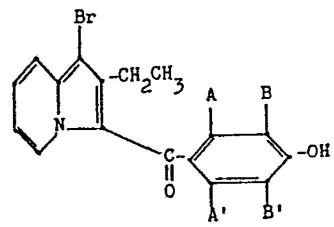

| Déplacements chimiques (en ppm) | | | | | | Constantes de couplages (en cps) |
|---|---|---|---|---|---|---|
| δ 1 | δ 5 | δ 6 | δ 7 | δ8 | | |
| – | 9,0 dt | 6,91 | 7,23 | 7,56 | AA′ = 7,56<br>BB′ = 6,90 | $J_{6,7}$ = 6,5<br>$J_{7,8}$ = 9<br>$J_{5,6}$ = 7 |

(solvant : $DMSOd_6$, référence : TMSc-a-d tétraméthylsilane)

De même, lorsqu'on utilise le procédé (C) au départ de (bromo-3 hydroxy-4 benzoyl)-3 indolizine substituée en position 2 de façon à obtenir un dérivé dibromé, le second atome de brome ainsi introduit dans la molécule, se fixe en position 1 du cycle indolizine : le spectre R.M.N. montre la présence d'un atome de brome en position 1 de l'indolizine (absence de proton $H_1$) et d'un atome de brome en position ortho du radical hydroxyle (la multiplicité et le déplacement chimique sont en accord avec une telle structure).

A titre d'exemple, les caractéristiques R.M.N. de la bromo-1 éthyl-2 (bromo-3 hydroxy-4 benzoyl)-3 indolizine sont les suivantes:

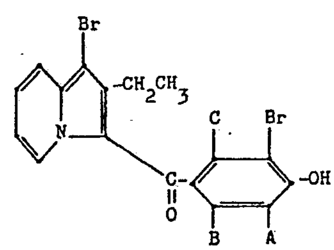

| Déplacements chimiques (en ppm) | | | | | | Constantes de couplages (en cps) |
|---|---|---|---|---|---|---|
| δ 1 | δ 5 | δ 6 | δ 7 | δ 8 | | |
| – | 9,18 dt | 6,96 m | 7,27 m | 7,54 | $H_C$ = 7,80 d<br>$H_B$ = 7,55 q<br>$H_A$ = 7,09 d | $J_{A,B}$ = 8,4 $J_{6,7}$ = 6,75<br>$J_{B,C}$ = 2,0 $J_{5,6}$ = 7,0<br>$J_{7,8}$ = 8,8 |

(solvant : $DMSOd_6$, référence : TMS)

Dans le cas des dérivés tribromés, la présence d'un singulet attribué au noyau p-hydroxybenzoyle implique que deux atomes de bromes sont situés en position ortho de la fonction hydroxyle.

A titre d'exemple, les caractéristiques R.M.N. de la bromo-1 éthyl-2 (dibromo-3,5 hydroxy-4 benzoyl)-3 indolizine sont les suivantes:

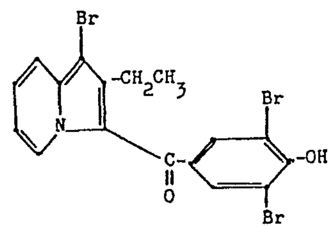

| Déplacements chimiques (en ppm) | | | | | | Constantes de couplages (en cps) |
|---|---|---|---|---|---|---|
| δ 1 | δ 5 | δ 6 | δ 7 | δ 8 | | |
| – | 9,28 dt | 7,01 m | 7,33 m | 7,77 dt | 7,81 s | $J_{5,6} = 7,0$<br>$J_{8,7} = 8,75$<br>$J_{5,6} = 6,5$ |

(solvant : $DMSOd_6$, référence : TMS)

Les composés de formule II sont soit des produits connus ayant été décrits dans le brevet britannique N° 1.518.443 ou des composés pouvant être préparés par la méthode décrite par GUBIN et coll., dans Eur. J. Med. Chem., 1977, *12*, 345.

Les composés de formule III peuvent être obtenus en faisant réagir le chlorure de thionyle avec un acide chloro-3 ou bromo-3 ou méthoxy-3 p-toluenesulfonyl-4 benzoïque. Les dérivés d'acide chloro- ou bromo- benzoïque en question sont des composés connus ayant été décrits par A.S. HUSSEY et coll. dans J. Am. Chem. Soc., *78*, 850 (1950) et dans le brevet N° 69.116 de la République Démocratique d'Allemagne tandis que le dérivé d'acide méthoxy-benzoïque est un produit commercial. Quant aux composés de formule V, ceux-ci peuvent être également obtenus selon des procédés connus. Par exemple, le chlorure de bromo-3 hydroxy-4 chloro-5 benzoyle est préparé en faisant réagir le chlorure de thionyle avec l'acide bromo-3 hydroxy-4 chloro-5 benzoïque lui-même obtenu au départ d'acide bromo-3 hydroxy-4 benzoïque et d'acide chlorhydrique concentré en présence de chlorate de potassium.

Les composés de formules VI et VII dans lesquels $X_2$ et $X_3$ sont chacun hydrogène sont des produits connus ayant été décrits dans le susdit brevet britannique alors que les autres composés de formules VI et VII sont soit des composés de l'invention soit des composés pouvant être préparés selon des procédés décrits ci-dessus.

Chez l'homme, l'acide urique est formé essentiellement par l'oxydation de l'hypoxanthine et de la xanthine catalysée par la xanthine oxydase. A l'heure actuelle, l'allopurinol constitue un des produits commerciaux les plus largement utilisés pour le traitement de l'hyperuricémie primaire de la goutte. Contrairement aux agents uricosuriques lesquels augmentent l'excrétion rénale d'urate, l'allopurinol inhibe les étapes ultimes de la biosynthèse de l'acide urique.

A de faibles concentrations, l'allopurinol dont la structure chimique est proche de celle de la xanthine et de l'hypoxanthine, constitue un excellent substrat pour une inhibition compétitive de la xanthine oxydase. L'oxypurinol, le métabolite formé par l'action de la xanthine oxydase sur l'allopurinol est un inhibiteur non compétitif extrêmement puissant du même enzyme. Par conséquent, l'activité inhibitrice en question dépend de la cinétique de deux réactions à savoir l'oxydation en oxypurinol d'une part et la décomposition du complexe enzyme-oxypurinol d'autre part.

L'inhibition des dernière et avant-dernière étapes de la biosynthèse de l'acide urique réduit la concentration plasmatique et l'excrétion urinaire d'acide urique et augmente la concentration plasmatique et l'excrétion rénale des précurseurs les plus solubles de l'oxypurine. L'allopurinol s'apparente aux bases puriques. De ce fait, il peut interférer dans un système de bases puriques autre que la xanthine, ce qui peut constituer une source de désordres physioligiques.

Pour cette raison, il est intéressant, que le thérapeute puisse disposer d'agents non apparentés aux bases puriques mais capables, néanmoins, d'exercer, comme l'allopurinol, une action inhibitrice sur la xanthine oxydase et par là de freiner la biosynthèse de l'acide urique. En outre, l'allopurinol présente une structure chimique relativement complexe, ce qui rend sa synthèse difficile.

Par conséquent, il est primordial de rechercher des agents plus faciles à préparer que l'allopurinol et qui soient capables d'exercer une action inhibitrice sur la xanthine oxydase.

Les dérivés d'indolizine de l'invention satisfont à ces exigences.

A cet effet, on peut citer les composés suivants:

Ethyl-2 (dibromo-3,5 hydroxy-4 benzoyl)-3 indolizine.
Bromo-1 n-propyl-2 (dibromo)-3,5 hydroxy-4 benzoyl)-3 indolizine.
Isopropyl-2 (dibromo-3,5 hydroxy-4 benzoyl)-3 indolizine.

On connait déjà des dérivés de benzofuranne, plus particulièrement des (dibromo-3,5 et diiodo-3,5 hydroxy-4 benzoyl)-3 benzofurannes, possédant une activité inhibitrice sur la xanthine oxydase [Arch. int. Pharmacodyn., 165, *25,* (1967)].

On a maintenant trouvé, dans le cadre de la présente invention, qu'il est possible d'exalter de manière considérable l'effet inhibiteur des dérivés de benzofuranne en question sur la xanthine oxydase et ce, en remplaçant l'entité benzofuranne par le cycle indolizine. Ainsi, on a observé que cette substitution fournit une série de nouveaux inhibiteurs de la xanthine oxydase, à savoir les indolizines de l'invention, certains d'entre eux étant dix à vingt fois plus actifs que les dérivés de benzofuranne en question.

On connait déjà des dérivés d'indolizine possédant des propriétés pharmacologiques. Par exemple, on a publié dans Eur. J. Med. Chem. cité ci-dessus, que certains dérivés de (dialkylamino-alkoxy-4 benzoyl)-3 indolizine possèdent des propriétés antiangineuses.

Cependant, aucune activité inhibitrice de la xanthine oxydase n'a été révélée, à l'heure actuelle pour des dérivés de la série des indolizines. Par conséquent, il n'était pas possible dans l'état présent des connaissances, de déduire que les composés de l'invention peuvent exercer une activité inhibitrice de la xanthine oxydase.

Comme mentionné précédemment, les composés de l'invention sont également doués d'une action inhibitrice de l'adénosine désaminase.

Des tests entrepris avec des dérivés de benzofuranne, notamment avec des dérivés décrits dans la référence extraite de Arch. int. Pharmacodyn. citée ci-dessus, ont montré que l'activité inhibitrice de ces dérivés de benzofuranne sur l'adénosine désaminase est nulle ou très faible.

L'existence d'une association entre une déficience génétique en adénosine désaminase et une immunoincompétence totale ouvre de nouvelles perspectives dans la recherche de la réponse immunitaire. Il semble, en effet, que l'adénosine et la désoxyadénosine (ou un de leurs métabolites tels que la déoxy-ATP) soient directement cytotoxiques pour les lymphocytes et inhibent la prolifération, la différenciation et la maturation des cellules immunocompétentes. Des inhibiteurs connus de l'adénosine désaminase par exemple le tétrahydro-3,4,7,8 β-D-ribofuranosyl-3 imidazo [4,5-d] [1,3] diazépinol-8 ou coformycine et l'érythro-9 (hydroxy-2 nonyl-3) adénine ou EHNA potentialisent cette toxicité. Par conséquent, les inhibiteurs de l'adénosine désaminase pourraient exercer une activité immuno-suppressive en augmentant les taux intracellulaires d'adénosine, ou d'un de ses métabolites, dans les lymphocites.

Contrairement aux immunosuppresseurs actuellement utilisés, les inhibiteurs de l'adénosine désaminase pourraient agir spécifiquement au niveau des cellules immunocompétentes et être relativement peu toxiques pour les autres types cellulaires. En effet, la conversion de l'adénosine en inosine, catalysée par l'adénosine désaminase, semble être une voie relativement mineure du métabolisme des purines dans la plupart des cellules somatiques.

Tout comme l'allopurinol dont question précédemment, l'EHNA s'apparente aux bases puriques. Par conséquent, l'EHNA pourrait également interférer avec d'autres enzymes du métabolisme des purines et engendrer des désordres physiologiques liés à cette absence de spécificité.

Des tests effectués avec des composés de l'invention comparativement à l'EHNA ont montré que les dérivés d'indolizine de formule I exercent une action plus spécifique que l'EHNA.

L'ensemble de ces propriétés rendront les dérivés d'indolizine de l'invention extrêmement valables comme inhibiteurs de l'adénosine désaminase. Ainsi, ces composés pourraient être largement utilisés en immunopathologie par exemple dans la prévention du phénomène de rejet lors de transplantations d'organes, dans les cas de maladies rhumatismales auto-immunes telles que la polyarthrite chronique évolutive et dans les cas de lupus érythémateux disséminé, de glomérulonéphrites auto-immunes, d'hépatites chroniques aggressives, de sclérose en plaques etc...

L'immunosuppression n'est pas la seule application thérapeutique potentielle des inhibiteurs d'adénosine désaminase.

Ces substances pourraient également montrer un effet cytotoxique spécifique dans les néoplasies lymphocitaires et également potentialiser l'activité d'argents anti-tumoraux ou antiviraux comme la cordycépine (ou désoxy-3' adénosine) ou l'adénine arabinoside.

"In vivo", l'adénine arabinoside (Ara-A) est convertie rapidement par désamination en un composé possédant des propriétés antivirales et anti-tumorales moins puissantes qu'elle à savoir l'hypoxanthine arabinoside. L'inhibition de cette étape par des inhibiteurs d'adénosine désaminase conduit à une potentialisation de l'activité antivirale et anti-tumorale de l'adénine arabinoside.

Des tests effectués "in vivo" ont démontré que les composés de l'invention peuvent agir comme inhibiteurs de la désamination de l'adénine arabinoside par l'adénosine désaminase donnant ainsi

naissance à une augmentation synergique de l'activité anti-herpétique de l'Ara-A chez la souris.

Comme inhibiteurs de l'adénosine désaminase, les composés suivants peuvent être considérés comme les dérivés préférés de l'invention:

Bromo-1 phényl-2 (bromo-3 hydroxy-4 benzoyl)-3 indolizine.
Bromo-1 (fluoro-4 phényl)-2 (bromo-3 hydroxy-4 benzoyl)-3 indolizine.
Méthoxy-1 phényl-2 (bromo-3 hydroxy-4 benzoyl)-3 indolizine.

Comme mentionné précédemment, on a également trouvé que des composés de l'invention potentialisent les effets cardiovasculaires des catécholamines, ce qui rend ces composés utiles dans le traitement, par exemple, de troubles du système vasculaire cérébral.

Au niveau cardiaque, cette potentialisation des effets des catécholamines se traduit par une augmentation de la contractilité du myocarde, ce qui pourrait être utile dans le traitement d'insuffisance cardiaque et d'angine de poitrine.

Dans cette perspective, on peut citer le composé suivant de formule I:
(Bromo-4 phényl)-2 (bromo-3 hydroxy-4 benzoyl)-3 indolizine.

Des tests ont été entrepris en vue de démontrer les effets pharmacologiques des composés de l'invention.

Ainsi, on a effectué des tests "in vitro" dans la cuvette d'absorption d'un spectrophotomètre U.V. en ce qui concerne l'effet inhibiteur sur la xanthine oxydase et l'adénosine désaminase. De même, on a pratique des tests "in vivo" en vue de mettre en évidence l'action uricosurique et l'effet inhibiteur sur l'adénosine désaminase.

I. *Inhibition de la xanthine oxydase*

Ce test a été effectué en utilisant la xanthine oxydase de lait. L'activité de la préparation enzymatique a été mesurée en suivant la formation d'acide urique au départ d'hypoxanthine en présence d'oxygène et d'une solution de tampon phosphate.

On a utilisé le processus expérimental décrit ci-après:

Dans la cellule d'absorption, on a introduit les solutions suivantes:

1,5 ml d'une solution de tampon phosphate 0,1 molaire (pH = 7,4) saturée en oxygène.

0,3 ml d'une solution 1 m molaire en tétraacétate d'éthylène diamine.

0,8 ml d'eau.

0,01 ml d'une solution commerciale de xanthine oxydase ayant une concentration connue en enzyme.

0,01 ml d'une solution éthanolique $3.10^{-3}$ molaire en composé à étudier.

Après une incubation de deux minutes, la réaction est enclenchée par addition de 0,3 ml d'une solution $10^{-3}$ molaire d'hypoxanthine. On enregistre alors par spectrophotométrie à 293 mm, la vitesse d'oxydation de l'hypoxanthine à 37°C. La densité optique est relevée automatiquement. On effectue des mesures semblables au moyen d'une solution contrôle contenant tous les ingrédients ci-dessus excepté le composé à étudier. De cette manière, on obtient d'une part pour la solution contenant l'inhibiteur, d'autre part pour la solution contrôle, une courbe représentant la variation de la densité optique en fonction du temps. On mesure la pente des deux courbes et on l'exprime en nombre de moles d'acide urique formé en une minute. Ainsi, il est possible de comparer les valeurs obtenues et de calculer, en pourcentage, l'inhibition de l'activité de la xanthine oxydase induite par un composé de l'invention. On a enregistré les résultats suivants:

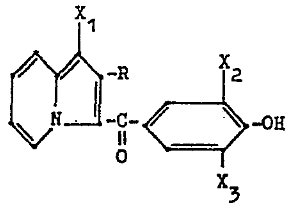

| R | $X_1$ | $X_2$ | $X_3$ | Inhibition de l'action de la xanthine oxydase in vitro (en %) à $10^{-5}$ M |
|---|---|---|---|---|
| Méthyle | H | H | Br | 74 |
| Isopropyle | H | H | Br | 94 |
| Ethyle | H | H | Cl | 87 |
| Isopropyle | H | H | Cl | 88 |
| Ethyle | H | H | $OCH_3$ | 57 |
| Bromo-4 phényle | H | H | Br | 22 |
| Bromo-3 phényle | H | H | Cl | 23 |
| Méthyle | H | Br | Br | 91 |
| Ethyle | H | Br | Br | 98 |
| Isopropyle | H | Br | Br | 99 |
| Bromo-4 phényle | H | Br | Br | 23 |
| Méthyle | H | Cl | Cl | 84 |
| Ethyle | H | Cl | Cl | 98 |
| Isopropyle | H | Cl | Cl | 96 |
| Méthyl-4 phényle | Br | H | H | 48 |
| n-Propyle | Br | H | Cl | 84 |
| Ethyle | Br | Br | H | 93 |
| n-Propyle | Br | Br | H | 97 |
| Méthoxy-4 phényle | Br | Br | H | 80 |
| n-Propyle | Br | Br | Br | 95 |
| Phényle | Br | Br | Br | 48 |
| Méthyle | Br | Cl | H | 44 |
| Ethyle | Cl | Br | H | 34 |
| Méthyle | Br | Cl | Cl | 46 |
| Ethyle | Cl | Br | Br | 64 |

A la concentration de $10^{-6}$ M, l'isopropyl-2 (dibromo-3,5 hydroxy-4 benzoyl)-3 indolizine exerce déjà 84% d'inhibition de l'action de la xanthine oxydase "in vitro".

II. *Action uricosurique*

L'action uricosurique a été déterminée chez l'être humain après administration d'une dose unique de 300 mg d'éthyle-2 (dibromo-3,5 hydroxy-4 benzoyl)-3 indolizine par voie orale.

L'excrétion urinaire d'acide urique s'accroit de 40% entre la 12 ème et la 24 ème heure après administration.

L'augmentation moyenne est de 22% par 24 heures.

Le volume urinaire par 24 heures n'est pas sensiblement modifié alors que l'uricémie est significativement diminuée de 9% à la 5 ème heure, moment où se situe le taux plasmatique le plus élevé en composé à étudier.

III. *Inhibition de l'adénosine désaminase*

A. *Test "in vitro"*

Ce test a été réalisé en utilisant de l'adénosine désaminase obtenue au départ d'intestin de veau.

On a utilisé le processus expérimental décrit ci-après:

Dans la cellule d'absorption, on a introduit les solutions suivantes:

2,7 ml d'une soluton tampon phosphate 0,1 molaire (pH = 7,4)

0,3 ml d'une solution $10^{-4}$ ou $5.10^{-4}$ molaire en adénosine dans la solution tampon phosphate (concentration finale en adénosine: $10^{-5}$ ou $5.10^{-5}$ mole)

0,01 ml d'une solution éthanolique $3.10^{-3}$ molaire en composé à étudier (environ 10 milliunités).

Après une incubation de deux minutes, la réaction enzymatique est enclenchée par addition de 0,03 ml d'une solution commerciale d'adénosine désaminase ayant une concentration connue en enzyme. On effectue la réaction à 30°C et on enregistre par spectrophotométrie à 265 nm la diminution de l'absorbance liée à la conversion de l'adénosine en inosine par l'adénosine désaminase.

La réaction enzymatique peut être représentée par la réaction suivante:

$$\text{Adénosine} + H_2O \xrightarrow{\text{adénosine désaminase}} \text{Inosine} + NH_3$$

On effectue des mesures semblables au moyen d'une solution contrôle contenant tous les ingrédients ci-dessus excepté le composé à étudier. On établit ainsi des courbes représentant la diminution de l'absorbance en fonction du temps d'une part pour la solution contenant l'inhibiteur et d'autre part pour la solution contrôle. On mesure la pente des deux courbes, on l'exprime en nombre de moles d'inosine formée en une minute puis on calcule le pourcentage d'inhibition de l'activité de l'adénosine désaminase induite par un composé de l'invention.

On a enregistré les résultats suivants:

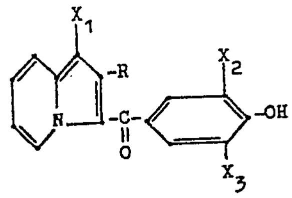

| R | $X_1$ | $X_2$ | $X_3$ | Inhibition de l'action de l'adénosine désaminase in vitro (en %) | |
|---|---|---|---|---|---|
| | | | | A $10^{-5}$ M d'adénosine | A $5.10^{-5}$ M d'adénosine |
| n-Butyle | H | H | Br | 60 | – |
| n-Propyle | H | H | Cl | 42 | – |
| Phényle | H | H | Br | 78 | – |
| Fluoro-4 phényle | H | H | Br | 60 | – |
| Phényle | H | H | Cl | 58 | – |
| Bromo-3 phényle | Cl | H | H | – | 55 |
| Phényle | Br | H | H | – | 52 |
| n-Butyle | Br | H | H | 45 | – |
| Fluoro-4 phényle | H | H | $OCH_3$ | – | 39 |
| Isopropyle | Br | Br | H | 75 | 54 |
| Phényle | Br | Br | H | 100 | 88 |
| Isopropyle | H | Cl | Cl | – | 76 |
| Phényle | Br | Cl | H | – | 86 |
| Fluoro-4 phenyle | Br | Br | H | 100 | 94 |
| Phényle | Cl | Cl | Cl | – | 57 |
| Isopropyle | Br | Br | Br | 57 | – |
| Phényle | Br | Br | Br | 53 | 22 |
| Phényle | $OCH_3$ | Cl | H | – | 82 |
| Phényle | $OCH_3$ | Br | H | – | 91 |

Des tests comparatifs pratiqués avec l'éthyl-2 (hydroxy-4 benzoyl)-3 benzofuranne et le phényl-2 (dibromo-3,5 hydroxy-4 benzoyl)-3 benzofuranne ont montré que ces deux composés provoquent respectivement une inhibition de 18% et de 15% de l'adénosine désaminase à la concentration de $10^{-5}$ M en adénosine.

Quant aux éthyl-2 ou isopropyl-2 ou n-butyl-2 (dibromo-3,5 hydroxy-4 benzoyl)-3 benzofurannes et à l'éthyl-2 (diiodo-3,5 hydroxy-4 benzoyl)-3 benzofuranne on a trouvé ces composés totalement inactifs comme inhibiteurs de l'adénosine désaminase à $10^{-5}$ M en adénosine.

A la concentration de $10^{-6}$ M, les bromo-1 phényl-2 et bromo-1 (fluoro-4 phényl)-2 (bromo-3 hydroxy-4 benzoyl)-3 indolizines exercent déjà respectivement 80% et 86% d'inhibition de l'adénosine désaminase "in vitro".

B. *Test "in vivo"*

Ce test a été effectué de façon à déterminer les effets de l'adénine arabinoside (Ara-A) et des composés de l'invention sur l'incidence des lésions herpétiques de la peau et la mortalité de souris nu/nu inoculées avec HSV-1 (KOS).

Des souris athymiques "nude" âgées de 20 à 25 jours sont inoculées par voie intracutanée avec HSV-1 (KOS) à environ $10^{4,5}$ PFU ("plaque-forming-units") par souris.

L'Ara-A et/ou un composé de l'invention est alors appliqué par voie topique à 1% dans le diméthylsulfoxide, 4 fois par jour aux jours 0, 1, 2, 3 et 4.

On enregistre alors le nombre de souris présentant une lésion épidermique (nécrose d'au moins 5 à 10 mm de longueur)) par nombre total de souris vivantes.

Les résultats sont consignés dans le dessin ci-joint dans lequel:

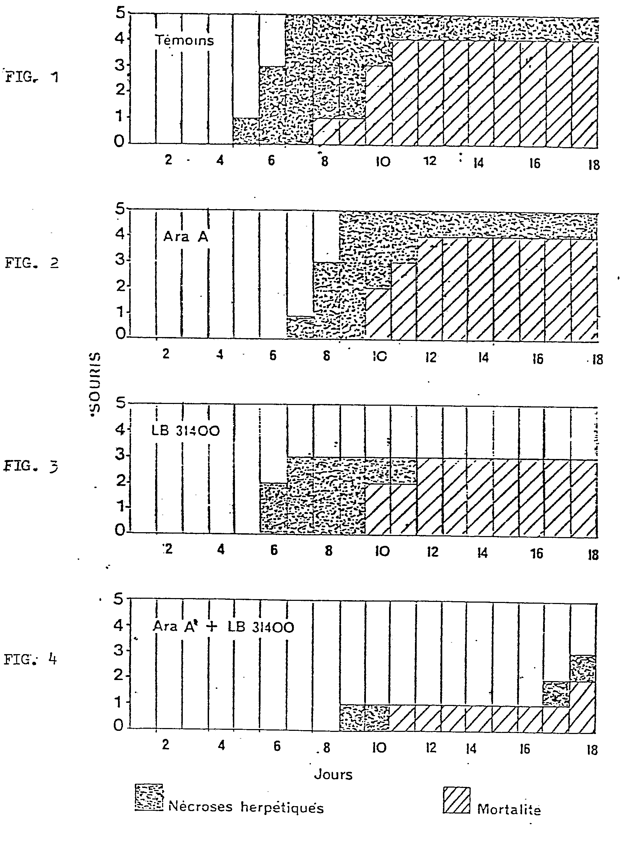

FIG. 1: représente les résultats obtenus avec les animaux témoins n'ayant reçu que le diméthylsulfoxyde
FIG. 2: représente les résultats obtenus avec l'Ara-A seul
FIG. 3: représente les résultats obtenus avec la bromo-1 phényl-2 (bromo-3 hydroxy-4 benzoyl)-3 indolizine dénommée LB 31400
FIG. 4: représente les résultats obtenus avec l'Ara-A en association avec LB 31400.

Ces résultats indiquent clairement que le composé de l'invention potentialise l'activité antivirale de l'adénine arabinoside.

IV. *Toxicité aiguë*

Des tests de toxicité aiguë ont été effectués chez la souris avec des composés de l'invention.

A la dose de 20 mg/kg de bromo-1 phényl-2 ou bromo-1 (fluoro-4 phényl)-2 (bromo-3 hydroxy-4 benzoyl)-3 indolizine, par voie intraveineuse, on n'a relevé aucune mortalité après 14 jours. Un résultat semblable a été obtenu suite à l'administration de 1500 mg/kg de bromo-1 isopropyl-2 ou éthyl-2 (dibromo-3,5 hydroxy-4 benzoyl)-3 indolizine par voie intragastrique.

Dans le cadre d'autres propriétés éventuelles que pourraient présenter les composés de l'invention, on attirera plus particulièrement l'attention sur l'activité inhibitrice de l'adénosine désaminase exercée par ces mêmes composés et qui est décrite précédemment.

Comme cette activité est également présentée par certains composés connus, par exemple la désoxy-2' coformycine et la coformycine qui possèdent des propriétés antiparasitaires, il est probable que les composés de l'invention eux-mêmes soient doués de propriétés antiparasitaires utiles en thérapeutique humaine ou vétérinaire.

Les compositions thérapeutiques de l'invention peuvent être présentées sous toute forme convenant à l'administration en thérapie humaine ou vétérinaire. Pour ce qui concerne l'unité d'administration, celle-ci peut prendre la forme, par exemple d'un comprimé, d'une dragée, d'une capsule, d'une gélule, d'une poudre, d'une suspension ou d'un sirop pour l'administration orale ou d'un suppositoire pour l'administration rectale.

Suivant la voie d'administration choisie, les compositions pharmaceutiques ou vétérinaires de l'invention seront préparées en associant au moins un composé de formule I avec un excipient approprié, ce dernier pouvant être constitué d'au moins un ingrédient sélectionné parmi les substances suivantes: lactose, amidons, talc, stéarate de magnésium, polyvinylpyrrolidone acide alginique et silice colloïdale.

Les Exemples non limitatifs suivants illustrent la préparation des composés de formule I et d'une composition les contenant:

Exemple 1

*Méthyl-2 (bromo-3 hydroxy-4 benzoyl)-3 indolizine*

A une solution de 101 g (0,73 mole) de méthyl-2 indolizine dans 900 ml de dichloréthane, on ajoute assez rapidement tout en maintenant la température entre 40 et 50°C, 284,5 g (0,73 mole) de chlorure de (bromo-3 tosyloxy)-4 benzoyle dans 900 ml de dichloréthane. On agite pendant 20 heures et on évapore à sec. On traite le résidu par un litre d'isopropanol pendant 2 heures au reflux, ce qui permet l'extraction d'impuretés. On laisse refroidir sous agitation, on essore et on lave avec 1,5 l d'isopropanol, ce qui fournit 210 g de produit brut cristallin. (rendement: 59,4%).

On chauffe au reflux pendant 20 heures le produit brut précédemment obtenu avec une solution de 175 ml d'hydroxyde de sodium à 30% en présence de 175 ml de méthanol. On laisse refroidir, on acidifie avec de l'acide chlorhydrique concentré et on essore. On lave jusqu'à neutralité et on recristallise dans le dichloréthane.

De cette manière, on obtient 98,8 g de méthyl-2 (bromo-3 hydroxy-4 benzoyl)-3 indolizine.
Rendement: 80,3%.
P.F.: 216—217°C.

A partir de produits appropriés et en utilisant le procédé décrit ci-dessus, on a préparé les composés suivants:

| Composé | P.F. °C |
|---|---|
| Ethyl-2 (bromo-3 hydroxy-4 benzoyl)-3 indolizine | 210 (dichloréthane) |
| n-Propyl-2 (bromo-3 hydroxy-4 benzoyl)-3 indolizine | 169 (dichloréthane) |
| Isopropyl-2 (bromo-3 hydroxy-4 benzoyl)-3 indolizine | 89 méthanol) |
| n-Butyl-2 (bromo-3 hydroxy-4 benzoyl)-3 indolizine | 167—168 (dichloréthane) |
| Phényl-2 (bromo-3 hydroxy-4 benzoyl)-3 indolizine | 219—220 (dioxane) |
| (Méthyl-4 phényl)-2 (bromo-3 hydroxy-4 benzoyl)-3 indolizine | 212—213 (dichloréthane) |
| (Fluoro-4 phényl)-2 (bromo-3 hydroxy-4 benzoyl)-3 indolizine | 227 (dichloréthane) |
| (Chloro-4 phényl)-2 (bromo-3 hydroxy-4 benzoyl)-3 indolizine | 240—241 (dioxane) |
| (Bromo-4 phényl)-2 (bromo-3 hydroxy-4 benzoyl)-3 indolizine | 235—236 (dichloréthane) |
| (Bromo-3 phényl)-2 (bromo-3 hydroxy-4 benzoyl)-3 indolizine | 199—200 (dichloréthane) |
| (Dichloro-3,4 phényl)-2 (bromo-3 hydroxy-4 benzoyl)-3 indolizine | 251 (dioxane) |

## Exemple 2

*n-Propyl-2 (bromo-3 hydroxy-4 benzoyl)-3 indolizine*

A une solution de 3,2 g (0,02 mole) de n-propyl-2 indolizine dans 20 ml de dichloréthane, on ajoute, à température ambiante, 8 g (0,02 mole) de chlorure de (chloro-3 tosyloxy)-4 benzoyle dans 80 ml de dichloréthane. On agite durant 20 heures et ou évapore ensuite à sec. On obtient ainsi 9,4 g d'un produit huileux que l'on chauffe au reflux pendant 4 heures avec une solution de 50 ml d'hydroxyde de sodium à 30% en présence de 50 ml de méthanol. On laisse refroidir, on acidifie avec de l'acide chlorhydrique concentré et on essore. On lave jusqu'à neutralité et on recristallise dans 10 ml de méthanol.

De cette manière, on obtient 1 g de n-propyl-2 (chloro-3 hydroxy-4 benzoyl)-3 indolizine. Rendement: 15,9%.

P.F.: 165°C.

A partir de produits appropriés et en utilisant le procédé décrit ci-dessus, on a préparé les composés suivants:

| Composé | P.F. °C |
|---|---|
| Méthyl-2 (chloro-3 hydroxy-4 benzoyl)-3 indolizine | 239—240 (dioxane) |
| Ethyl-2 (chloro-3 hydroxy-4 benzoyl)-3 indolizine | 208—209 (dioxane) |
| isopropyl-2 (chloro-3 hydroxy-4 benzoyl)-3 indolizine | 161—162 (méthanol) |
| n-Butyl-2 (chloro-3 hydroxy-4 benzoyl)-3 indolizine | 151—152 (benzène) |

| Composé | P.F. °C |
|---|---|
| Phényl-2 (chloro-3 hydroxy-4 benzoyl)-3 indolizine | 210—211 (dichloréthane) |
| (Méthyl-4 phényl)-2 (chloro-3 hydroxy-4 benzoyl)-3 indolizine | 215—216 (dioxane) |
| (Bromo-3 phényl)-2 (chloro-3 hydroxy-4 benzoyl)-3 indolizine | 181—182 (dichloréthane) |
| (Chloro-4 phényl)-2 (chloro-3 hydroxy-4 benzoyl)-3 indolizine | 240—241 (dioxane) |
| (Bromo-4 phényl)-2 (chloro-3 hydroxy-4 benzoyl)-3 indolizine | 232—233 (dioxane) |

Exemple 3

*Méthoxy-1 phényl-2 (bromo-3 hydroxy-4 benzoyl)-3 indolizine*

A une solution de 19,4 g (0,09 mole) de méthoxy-1 phényl-2 indolizine dans 100 ml de dichloréthane, on ajoute, à température ambiante, 33,9 g (0,09 mole) de chlorure de (bromo-3 tosyloxy)-4 benzoyle dans 100 ml de dichloréthane. On agite durant 20 heures et on évapore ensuite à sec. On obtient ainsi un produit cristallin que l'on recristallise dans 250 ml d'isopropanol, ce qui donne 25,4 g d'un produit pur (P.F.: 153°C). On chauffe au reflux pendant 4 heures le produit ainsi obtenu et ce, avec 100 ml d'une solution d'hydroxyde de sodium à 30% et en présence de 50 ml de méthanol. On laisse refroidir, on acidifie avec de l'acide chlorhydrique concentré et on essore. On lave jusqu'à neutralité et on recristallise dans un mélange 1/1 de méthanol/chloroforme.

De cette manière, on obtient 14,6 g de méthoxy-1 phényl-2 (bromo-3 hydroxy-4 benzoyl)-3 indolizine.

Rendement: 78,5%.

P.F.: 205°C.

A partir de produits appropriés, et en utilisant le procédé décrit ci-dessus, on a préparé les composés suivants:

| Composé | P.F. °C |
|---|---|
| Méthoxy-1 phényl-2 (hydroxy-4 benzoyl)-3 indolizine | 199 (méthanol) |
| Méthoxy-1 phényl-2 (chloro-3 hydroxy-4 benzoyl)-3 indolizine | 203 (isopropanol) |
| Méthoxy-1 phényl-2 (méthoxy-3 hydroxy-4 benzoyl)-3 indolizine | 180 (méthanol) |
| Méthyl-2 (méthoxy-3 hydroxy-4 benzoyl)-3 indolizine | 182 (isopropanol) |
| Ethyl-2 (méthoxy-3 hydroxy-4 benzoyl)-3 indolizine | 157 (isopropanol) |
| (Fluro-4 phényl)-2 (méthoxy-3 hydroxy-4 benzoyl)-3 indolizine | 166 (benzène) |
| Bromo-1 méthyl-2 (méthoxy-3 hydroxy-4 benzoyl)-3 indolizine | 155 (méthanol) |
| Bromo-1 éthyl-2 (méthoxy-3 hydroxy-4 benzoyl)-3 indolizine | 160 (méthanol) |

Exemple 4

*Ethyl-2 (dibromo-3,5 hydroxy-4 benzoyl)-3 indolizine*

A une solution de 78,8 g (0,54 mole) d'éthyl-2 indolizine dans 1500 ml de dichloréthane, on

ajoute rapidement, 180 g (0,57 mole) de chlorure de dibromo-3,5 hydroxy-4 benzoyle (P.F.: 98—100°C).

On agite durant 24 heures à température ambiante et on laisse ensuite au repos pendant 24 heures. On filtre et on lave le produit obtenu sur filtre avec du dichloréthane et ensuite avec de l'éthanol jusqu'à obtention d'un filtrat jaune.

On obtient ainsi 112,1 g d'un solide cristallin jaune qui, recristallisé dans 2000 ml de dichloréthane donne 84 g de produit désiré.

P.F.: 213°C.

On obtient un second jet en concentrant le filtrat et les eaux de lavage jusqu'à environ 500 ml. Après repos, on filtre et on traite le produit ainsi obtenu sur filtre comme décrit précédemment. On obtient ainsi 26 g de produit brut, qui recristallisé dans le dichloréthane, fournit 23,9 g de produit désiré.

De cette manière, on obtient 107,9 g d'éthyl-2 (dibromo-3,5 hydroxy-4 benzoyl)-3 indolizine. Rendement: 47,2%.

P.F.: 213°C.

A partir des produits appropriés et en utilisant le procédé decrit ci-dessus, on a préparé les composés suivants:

| Composé | P.F. °C |
|---|---|
| Méthyl-2 (dibromo-3,5 hydroxy-4 benzoyl)-3 indolizine | 210 (éthanol) |
| n-Propyl-2 (dibromo-3,5 hydroxy-4 benzoyl)-3 indolizine | 143 (isopropanol/eau) |
| Isopropyl-2 (dibromo-3,5 hydroxy-4 benzoyl)-3 indolizine | 198 (éthanol) |
| n-Butyl-2 (dibromo-3,5 hydroxy-4 benzoyl)-3 indolizine | 171 (éthanol) |
| Phényl-2 (dibromo-3,5 hydroxy-4 benzoyl)-3 indolizine | 189 (éthanol-eau) |
| (Méthyl-4 phényl)-2 (dibromo-3,5 hydroxy-4 benzoyl)-3 indolizine. Sel sodique | 255 (pâteux) (eau) |
| (Méthoxy-4 phényl)-2 (dibromo-3,5 hydroxy-4 benzoyl)-3 indolizine | 192 (isopropanol/eau) |
| (Fluoro-4 phényl)-2 (dibromo-3,5 hydroxy-4 benzoyl)-3 indolizine. Sel sodique | 250 (pâteux) (eau) |
| (Chloro-4 phényl)-2 (dibromo-3,5 hydroxy-4 benzoyl)-3 indolizine | 139 (heptane/benzène) |
| (Dichloro-3,4 phényl)-2 (dibromo-3,5 hydroxy-4 benzoyl)-3 indolizine | 186 (heptane/benzène) |
| (Bromo-2 phényl)-2 (dibromo-3,5 hydroxy-4 benzoyl)-3 indolizine | 210 (heptane/benzène) |
| (Bromo-3 phényl)-2 (dibromo-3,5 hydroxy-4 benzoyl)-3 indolizine | 200 (heptane/benzène) |
| (Bromo-4 phényl)-2 (dibromo-3,5 hydroxy-4 benzoyl)-3 indolizine | 164 (heptane/benzène) |

Exemple 5

*Ethyl-2 (dibromo-3,5 hydroxy-4 benzoyl)-3 indolizine*

A une solution de 10 g (0,068 mole) d'éthyl-2 indolizine dans 250 ml de dichloréthane, on ajoute rapidement 15,8 g (0,07 mole) de chlorure de dichloro-3,5 hydroxy-4 benzoyle (P.F.: 92°C). On agite durant 24 heures à température ambiante et on chasse ensuite le solvant sous pression réduite. On

purifie alors le produit obtenu par chromatographie d'élution sur silice, l'éluant étant le chloroforme.

De cette manière, on obtient 11,5 g d'un solide jaune qui recristallisé dans 300 ml de dichloréthane donne 8,4 g d'éthyl-2 (dichloro-3,5 hydroxy-4 benzoyl)-3 indolizine.

Rendement: 37%.

P.F.: 206°C.

A partir des produits appropriés et en utilisant le procédé décrit ci-dessus, on a préparé les composés suivants:

| Composé | P.F. °C |
|---|---|
| Méthyl-2 (dichloro-3,5 hydroxy-4 benzoyl)-3 indolizine | 190 (dichloréthane/heptane 80/20) |
| n-Propyl-2 (dichloro-3,5 hydroxy-4 benzoyl)-3 indolizine | 140 (dichloréthane/heptane 70/30) |
| Isopropyl-2 (dichloro-3,5 hydroxy-4 benzoyl)-3 indolizine | 100 (dichloréthane) |
| Phényl-2 (dichloro-3,5 hydroxy-4 benzoyl)-3 indolizine | 193 (isopropanol) |
| (Bromo-4 phényl)-2 (dichloro-3,5 hydroxy-4 benzoyl)-3 indolizine | 202 (isopropanol) |

Exemple 6

*Bromo-1 éthyl-2 (hydroxy-4 benzoyl)-3 indolizine*

A un mélange de 5,3 g (0,02 mole) d'éthyl-2 (hydroxy-4 benzoyl)-3 indolizine, 5,6 (0,041 mole) d'acétate de sodium trihydraté et 50 ml d'acide acétique, on ajoute goutte à goutte en 3 h 3/4, 3.25 g (0,021 mole) de brome dans 50 ml d'acide acétique. Durant cette opération, on maintient la température entre 20 et 22°C. On agite encore pendant 3/4 heure et on ajoute ensuite 150 ml d'eau. On agite à nouveau pendant 1 heure et on essore le précipité formé.

Après réempâtage dans 50 ml puis dans 100 ml de benzène, il reste 6,35 g d'un solide que l'on recristallise dans 90 ml de méthanol.

De cette manière, on obtient 4,4 g de bromo-1 éthyl-2 (hydroxy-4 benzoyl)-3 indolizine.

Rendement: 63,9%

P.F.: 160—161,5°C.

A partir de produits appropriés et en utilisant le precédé décrit ci-dessus, on a préparé les composés suivants:

| Composé | P.F. °C |
|---|---|
| Bromo-1 méthyl-2 (hydroxy-4 benzoyl)-3 indolizine | 238—239 (dichloréthane) |
| Bromo-1 n-butyl-2 (hydroxy-4 benzoyl)-3 indolizine | 160—162 (benzène) |
| Bromo-1 n-pentyl-2 (hydroxy-4 benzoyl)-3 indolizine | 155—157 (dichloréthane) |
| Bromo-1 n-hexyl-2 (hydroxy-4 benzoyl)-3 indolizine | 162—163 (dichloréthane) |
| Bromo-1 n-heptyl-2 (hydroxy-4 benzoyl)-3 indolizine | 145—149 (benzène) |
| Bromo-1 n-octyl-2 (hydroxy-4 benzoyl)-3 indolizine | 132—133 (dichloréthane) |
| Bromo-1 phényl-2 (hydroxy-4 benzoyl)-3 indolizine | 210 (dioxane) |

| Composé | P.F. °C |
|---|---|
| Bromo-1 (méthyl-4 phényl)-2 (hydroxy-4 benzoyl)-3 indolizine | 197—198 (dichloréthane) |
| Bromo-1 (bromo-4 phényl)-2 (hydroxy-4 benzoyl)-3 indolizine | 220—225 (benzène) |
| Bromo-1 (bromo-3 phényl)-2 (hydroxy-4 benzoyl)-3 indolizine | 225 (tétrachlorure de carbone) |
| Bromo-1 (chlor-3 méthyl-4 phényl)-2 (hydroxy-4 benzoyl)-3 indolizine | 210 (benzène) |
| Bromo-1 (dichloro-3,4 phényl)-2 (hydroxy-4 benzoyl)-3 indolizine | 240 (dichloréthane) |

Exemple 7

*Chloro-1 éthyl-2 (hydroxy-4 benzoyl)-3 indolizine*

A une solution de 50,4 g (0,12 mole) d'éthyl-2 (tosyloxy-4 benzoyl)-3 indolizine dans 1100 ml de dichloréthane, on ajoute par portions en 2 heures, 26,8 g (0,2 mole) de N-chlorosuccinimide.

Durant cette opération, on maintient la température entre 0 et 10°C. On agite encore une heure et on lave la solution dichloréthanique avec 500 ml d'eau. On distille le solvant sous vide et on recristallise dans 500 ml de méthanol.

De cette façon, on obtient 20,65 g d'un produit que l'on chauffe au reflux pendant 4 heures en présence de 45 ml d'eau, 5,4 g d'hydroxyde de sodium et 50 ml d'isopropanol. On refroidit à 20°C, on acidifie avec 10 ml d'acide chlorhydrique concentré et on agite pendant environ 12 heures. On essore le précipité formé, ce qui fournit 12,7 g de produit désiré que l'on recristallise dans 400 ml de méthanol.

De cette manière, on obtient 10,8 g de chloro-1 éthyl-2 (hydroxy-4 benzoyl)-3 indolizine.
Rendement: 30%
P.F.: 179°C.

A partir de produits appropriés et en utilisant le procédé décrit ci-dessus, on a préparé les composés suivants:

| Composé | P.F. °C |
|---|---|
| Chloro-1 méthyl-2 (hydroxy-4 benzoyl)-3 indolizine | 212 |
| Chloro-1 n-propyl-2 (hydroxy-4 benzoyl)-3 indolizine | 165 (benzène-cyclohexane 1/1) |
| Chloro-1 n-butyl-2 (hydroxy-4 benzoyl)-3 indolizine | 156 (benzène) |
| Chloro-1 (bromo-3 phényl)-2 (hydroxy-4 benzoyl)-3 indolizine | 201—202 (isopropanol) |
| Chloro-1 (chloro-4 phényl)-2 (hydroxy-4 benzoyl)-3 indolizine | 249—250 (dioxane) |
| Chloro-1 (bromo-4 phényl)-2 (hydroxy-4 benzoyl)-3 indolizine | 237—238 (dioxane) |

Exemple 8

*Bromo-1 éthyl-2 (bromo-3 hydroxy-4 benzoyl)-3 indolizine*

A un mélange de 1,3 g (0,004 mole) d'éthyl-2 (bromo-3 hydroxy-4 benzoyl)-3 indolizine, 0,6 g (0,09 mole) d'acétate de sodium anhydre et 10 ml d'acide acétique, on ajoute goutte à goutte et sous agitation, en 3 h 3/4, 0,6 g (0,004 mole) de brome dans 10 ml d'acide acétique. Durant cette opération, on maintient la température entre 20 et 22°C. On agite encore 3/4 heure et on verse sur 50 ml d'eau. On essore le précipité formé et on le lave jusqu'à neutralité.

De cette manière, on obtient 1,4 g d'un solide qui après recristallisation dans 20 ml de chloroforme fournit 0,7 g de bromo-1 éthyl-2 (bromo-3 hydroxy-4 benzoyl)-3 indolizine.

Rendement: 45%
P.F.: 213—214°C.

A partir des produits appropriés et en utilisant le procédé décrit ci-dessus, on a préparé les composés suivants:

| Composé | P.F. °C |
|---|---|
| Bromo-1 méthyl-2 (bromo-3 hydroxy-4 benzoyl)-3 indolizine | 245—246 (dichloréthane) |
| Bromo-1 n-propyl-2 (bromo-3 hydroxy-4 benzoyl)-3 indolizine | 189—191 (benzène) |
| Bromo-1 isopropyl-2 (bromo-3 hydroxy-4 benzoyl)-3 indolizine | 130—132 (benzène) |
| Bromo-1 n-butyl-2 (bromo-3 hydroxy-4 benzoyl)-3 indolizine | 183—184 (benzène) |
| Bromo-1 n-pentyl-2 (bromo-3 hydroxy-4 benzoyl)-3 indolizine | 174—175 (dichloréthane) |
| Bromo-1 n-hexyl-2 (bromo-3 hydroxy-4 benzoyl)-3 indolizine | 175—177 (dichloréthane) |
| Bromo-1 n-heptyl-2 (bromo-3 hydroxy-4 benzoyl)-3 indolizine | 165—166 (dichloréthane) |
| Bromo-1 n-octyl-2 (bromo-3 hydroxy-4 benzoyl)-3 indolizine | 161,5—163 (dichloréthane) |
| Bromo-1 phényl-2 (bromo-3 hydroxy-4 benzoyl)-3 indolizine | 184,5—186 (benzène) |
| Bromo-1 (méthyl-4 phényl)-2 (bromo-3 hydroxy-4 benzoyl)-3 indolizine | 231 (dichloréthane) |
| Bromo-1 (méthoxy-4 phényl)-2 (bromo-3 hydroxy-4 benzoyl)-3 indolizine | 204—205 (dichloréthane) |
| Bromo-1 (fluoro-4 phényl)-2 (bromo-3 hydroxy-4 benzoyl)-3 indolizine | 196 (benzène) |
| Bromo-1 (chloro-4 phényl)-2 (bromo-3 hydroxy-4 benzoyl)-3 indolizine | 228—230 (benzène) |
| Bromo-1 (bromo-4 phényl)-2 (bromo-3 hydroxy-4 benzoyl)-3 indolizine | 240—245 (dichloréthane) |
| Bromo-1 (bromo-3 phényl)-2 (bromo-3 hydroxy-4 benzoyl)-3 indolizine | 185—186 (tétrachlorure de carbone) |
| Bromo-1 (bromo-2 phényl)-2 (bromo-3 hydroxy-4 benzoyl)-3 indolizine | 213—214 (benzène) |
| Bromo-1 (chloro-3 méthyl-4 phényl)-2 (bromo-3 hydroxy-4 benzoyl)-3 indolizine | 201—202 (dichloréthane) |
| Bromo-1 (dichloro-3,4 phényl)-2 (bromo-3 hydroxy-4 benzoyl)-3 indolizine | 207—209 (benzène) |
| Bromo-1 méthyl-2 (chloro-3 hydroxy-4 benzoyl)-3 indolizine | 228 (dioxane) |

| Composé | P.F. °C |
|---|---|
| Bromo-1 éthyl-2 (chloro-3 hydroxy-4 benzoyl)-3 indolizine | 191—192 (dichloréthane) |
| Bromo-1 n-propyl-2 (chloro-3 hydroxy-4 benzoyl)-3 indolizine | 180—181 (isopropanol) |
| Bromo-1 isopropyl-2 (chloro-3 hydroxy-4 benzoyl)-3 indolizine | 174 (benzène) |
| Bromo-1 n-butyl-2 (chloro-3 hydroxy-4 benzoyl)-3 indolizine | 177—178 (dichloréthane) |
| Bromo-1 phényl-2 (chloro-3 hydroxy-4 benzoyl)-3 indolizine | 198—199 (benzène) |
| Bromo-1 (chloro-4 phényl)-2 (chloro-3 hydroxy-4 benzoyl)-3 indolizine | 212—213 (dichloréthane) |
| Bromo-1 (bromo-3 phényl)-2 (chloro-3 hydroxy-4 benzoyl)-3 indolizine | 126—128 (benzène) |
| Bromo-1 (bromo-4 phényl)-2 (chloro-3 hydroxy-4 benzoyl)-3 indolizine | 231—232 (dichloréthane) |

Exemple 9

*Chloro-1 éthyl-2 (bromo-3 hydroxy-4 benzoyl)-3 indolizine*

A une solution de 4,98 g (0,01 mole) d'éthyl-2 (bromo-3 tosyloxy-4 benzoyl)-3 indolizine dans 100 ml de dichloréthane, on ajoute, par petites fractions et sous bonne agitation, 1,98 g (0,015 mole) de N-chlorosuccinimide. On maintient la température entre 19 et 22°C au cours de l'introduction. On agite pendant 2 heures à température ambiante et on chasse ensuite le solvant sous pression réduite. Au produit brut ainsi obtenu, on ajoute 50 ml d'acétone et 100 ml de méthanol puis on agite durant 1 heure afin de solubiliser les impuretés présentes.

Après essorage et lavage avec du méthanol, on obtient 2,05 g de produit que l'on chauffe à reflux pendant 20 heures avec une solution de 10 ml d'hydroxyde de sodium à 30% en présence de 10 ml de méthanol. On laisse refroidir, on acidifie avec de l'acide chlorhydrique concentré, on essore et on lave jusqu'à neutralité.

De cette manière, on obtient 0,97 g de chloro-1 éthyl-2 (bromo-3 hydroxy-4 benzoyl)-3 indolizine.

Rendement: 66,9%

P.F.: 204°C.

A partir de produits appropriés et en utilisant le procédé décrit ci-dessus, on a préparé les composés suivants:

| Composé | P.F. °C |
|---|---|
| Chloro-1 éthyl-2 (chloro-3 hydroxy-4 benzoyl)-3 indolizine | 183—184 (benzène) |
| Chloro-1 isopropyl-2 (chloro-3 hydroxy-4 benzoyl)-3 indolizine | ±93 |
| Chloro-1 phényl-2 (chloro-3 hydroxy-4 benzoyl)-3 indolizine | 192—193 (isopropanol) |
| Chloro-1 (bromo-3 phényl)-2 (chloro-3 hydroxy-4 benzoyl)-3 indolizine | 157,5—158,5 (acétate d'éthyle) |

Exemple 10

*Bromo-1 éthyl-2 (dibromo-3,5 hydroxy-4 benzoyl)-3 indolizine*

A une solution de 4,2 g (0,01 mole) d'éthyl-2 (dibromo-3,5 hydroxy-4 benzoyl)-3 indolizine dans 100 ml de dioxane, on ajoute 1,6 g (0,02 mole) d'acétate de sodium anhydre. Sous bonne agitation, on

ajoute ensuite goutte à goutte 1,6 g (0,01 mole) de brome dans 20 ml de dioxane. On maintient la température entre 20 et 22°C durant l'introduction.

Après une heure d'agitation supplémentaire, on coule le produit de réaction dans 250 ml d'eau. Il y a formation d'un précipité jaune-vert que l'on filtre et lave à l'eau jusqu'à neutralité du filtrat. Après sèchage sous vide on obtient 5,2 g de produit que l'on recristallise dans 50 ml de dichloréthane.

De cette manière, on obtient 4,2 g de bromo-1 éthyl-2 (dibromo-3,5 hydroxy-4 benzoyl)-3 indolizine après traitement au charbon actif.

Rendement: 83,6%

P.F.: 195°C.

A partit de produits appropriés et en utilisant le procédé déctrit ci-dessus, on a préparé les composés suivants:

| Composé | P.F. °C |
|---|---|
| Bromo-1 méthyl-2 (dibromo-3,5 hydroxy-4 benzoyl)-3 indolizine | 254—255 (dichloréthane) |
| Bromo-1 n-propyl-2 (dibromo-3,5 hydroxy-4 benzoyl)-3 indolizine | 183—184 (benzène) |
| Bromo-1 isopropyl-2 (dibromo-3,5 hydroxy-4 benzoyl)-3 indolizine | 188—189 (tétrachlorure de carbone) |
| Bromo-1 n-butyl-2 (dibromo-3,5 hydroxy-4 benzoyl)-3 indolizine | 162—163 (heptane) |
| Bromo-1 n-pentyl-2 (dibromo-3,5 hydroxy-4 benzoyl)-3 indolizine | 139—140 (tétrachlorure de carbone) |
| Bromo-1 n-hexyl-2 (dibromo-3,5 hydroxy-4 benzoyl)-3 indolizine | 152—153 (benzène) |
| Bromo-1 n-heptyl-2 (dibromo-3,5 hydroxy-4 benzoyl)-3 indolizine | 167—168 (benzène) |
| Bromo-1 n-octyl-2 (dibromo-3,5 hydroxy-4 benzoyl)-3 indolizine | 153—154 (benzène) |
| Bromo-1 phényl-2 (dibromo-3,5 hydroxy-4 benzoyl)-3 indolizine | 211—212 (benzène) |
| Bromo-1 (méthyl-4 phényl)-2 (dibromo-3,5 hydroxy-4 benzoyl)-3 indolizine | 148—150 (cyclohexane/tétrachlorure de carbone) |
| Bromo-1 (méthoxy-4 phényl)-2 (dibromo-3,5 hydroxy-4 benzoyl)-3 indolizine | 167—168 (isopropanol) |
| Bromo-1 (fluoro-4 phényl)-2 (dibromo-3,5 hydroxy-4 benzoyl)-3 indolizine | 191—192 (benzène) |
| Bromo-1 (chloro-4 phényl)-2 (dibromo-3,5 hydroxy-4 benzoyl)-3 indolizine | 203—204 (tétrachlorure de carbone) |
| Bromo-1 (bromo-4 phényl)-2 (dibromo-3,5 hydroxy-4 benzoyl)-3 indolizine | 203—204 (tétrachlorure de carbone) |
| Bromo-1 (bromo-3 phényl)-2 (dibromo-3,5 hydroxy-4 benzoyl)-3 indolizine | 269—270 (dichloréthane) |
| Bromo-1 (bromo-2 phényl)-2 (dibromo-3,5 hydroxy-4 benzoyl)-3 indolizine | 176—177 (tétrachlorure de carbone) |
| Bromo-1 (chloro-3 méthyl-4 phényl)-2 (dibromo-3,5 hydroxy-4 benzoyl)-3 indolizine | 214—215,5 (dichloréthane) |

| Composé | P.F. °C |
|---|---|
| Bromo-1 (dichloro-3,4 phényl)-2 (dibromo-3,5 hydroxy-4 benzoyl)-3 indolizine | 244 (benzène) |

Exemple 11

*Bromo-1 éthyl-2 (dibromo-3,5 hydroxy-4 benzoyl)-3 indolizine*

A un mélange de 5,3 g (0,02 mole) d'éthyl-2 (hydroxy-4 benzoyl)-3 indolizine, 17,7 g (0,13 mole) d'acétate de sodium trihydraté et 50 ml d'acide acétique, on ajoute goutte à goutte en 3 heures, 10,55 g (0,066 mole) de brome dans 50 ml d'acide acétique. Durant cette opération, on maintient la température entre 20 et 22°C. On continue l'agitation pendant 18 heures. On essore le précipité et on lave avec de l'acide acétique et de l'eau. On ajoute 450 ml d'eau au filtrat et on essore à nouveau. On réunit les deux précipités et on recristallise dans le tétrachlorure de carbone.

De cette manière, on obtient 7,9 g de bromo-1 éthyl-2 (dibromo-3,5 hydroxy-4 benzoyl)-3 indolizine.

Rendement: 79%.

Exemple 12

*Chloro-1 éthyl-2 (dibromo-3,5 hydroxy-4 benzoyl)-3 indolizine*

A une solution de 4,2 (0,01 mole) d'éthyl-2 (dibromo-3,5 hydroxy-4 benzoyl)-3 indolizine dans 300 ml de dichloréthane, on ajoute par petites fractions et sous bonne agitation, 1,8 g (0,015 mole) de N-chlorosuccinimide.

On maintient la température entre 15 et 20°C au cours de l'introduction. On agite durant 24 heures à température ambiante et on chasse ensuite le solvant sous pression réduite. On purifie le produit brut ainsi obtenu par chromatographie d'élution sur silice, l'éluant étant le chloroforme.

De cette manière, on obtient 2,3 g de chloro-1 éthyl-2 (dibromo-3,5 hydroxy-4 benzoyl)-3 indolizine après recristallisation dans un mélange 70/30 de dichloréthane/heptane.

Rendement: 50%

P.F.: 194°C.

A partir des produits appropriés et en utilisant le procédé décrit ci-dessus, on a préparé les composés suivants:

| Composé | P.F. °C |
|---|---|
| Chloro-1 phényl-2 (dibromo-3,5 hydroxy-4 benzoyl)-3 indolizine | 207 (heptane/dichloréthane 30/70) |
| Chloro-1 isopropyl-2 (bromo-3,5 hydroxy-4 chloro-5 benzoyl)-3 indolizine | 192—193 (dichloréthane) |
| Chloro-1 éthyl-2 (dichloro-3,5 hydroxy-4 benzoyl)-3 indolizine | 145 (heptane/dichloréthane 50/50) |
| Chloro-1 phényl-2 (dichloro-3,5 hydroxy-4 benzoyl)-3 indolizine | 228 (isopropanol) |

Exemple 13

*Bromo-1 méthyl-2 (dichloro-3,5 hydroxy-4 benzoyl)-3 indolizine*

A une solution de 3,2 g (0.01 mole) de méthyl-2 (dichloro-3,5 hydroxy-4 benzoyl)-3 indolizine dans 100 ml de dioxane, on ajoute 1,6 g (0,02 mole) d'acétate de sodium anhydre. Sous bonne agitation, on ajoute goutte à goutte et à 20°C 1,6 g (0,01 mole) de brome. On maintient la température entre 20 et 22°C durant l'introduction.

On agite une heure supplémentaire puis on verse dans 250 ml d'eau. On filtre le précipité jaune-vert ainsi formé puis on le lave avec de l'eau jusqu'à neutralité du filtrat. Après sèchage sous vide, on obtient un produit brut que l'on recristallise dans un mélange 80/20 de dichloréthane/heptane.

De cette manière, on obtient 1,7 g de bromo-1 méthyl-2 (dichloro-3,5 hydroxy-4 benzoyl)-3 indolizine après traitement au charbon actif.

Rendement: 42,6%

P.F.: 240°C.

A partir des produits appropriés et en utilisant le procédé décrit ci-dessus on a préparé les composés suivants:

| Composé | P.F. °C |
| --- | --- |
| Bromo-1 éthyl-2 (dichloro-3,5 hydroxy-4 benzoyl)-3 indolizine | 174 (dichloréthane/heptane 90/10) |
| Bromo-1 phényl-2 (dichloro-3,5 hydroxy-4 benzoyl)-3 indolizine | 236 (dichloréthane/heptane 90/10) |

Exemple 13

On a préparé une unité pharmaceutique pour l'administration orale en introduisant, suivant des techniques connues, 300 mg d'éthyl-2 (dibromo-3,5 hydroxy-4 benzoyl)-3 indolizine dans une gélule.

**Revendications**

1. Dérivés d'indolizine correspondant à la formule générale:

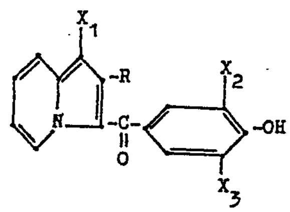

dans laquelle R représente un radical alkyle, linéaire ou ramifié, ayant de 1 à 8 atomes de carbone, un groupement phényle ou un groupement phényle portant un ou deux substituants, identiques ou différents choisis parmi des atomes d'halogène et parmi des groupements alkyles inférieurs et alkoxy inférieurs et $X_1$, $X_2$ et $X_3$, qui sont identiques ou différents, représentent chacun hydrogène, chlore, brome ou méthoxy, à condition que lorsqu'ils sont identiques $X_1$, $X_2$ et $X_3$ ne sont pas simultanément hydrogène.

2. Dérivés d'indolizine selon la Revendication 1 dans laquelle les atomes d'halogène sont des atomes de fluor, de chlore ou de brome et les groupements alkyles inférieurs et alkoxy inférieurs sont les groupements méthyle et méthoxy.

3. Dérivés d'indolizine selon la Revendication 1 dans laquelle R représente un radical alkyle, linéaire ou ramifié, ayant de 1 à 8 atomes de carbone, un radical phényle, un radical monofluoro-, monochloro-, monobromo-, monométhyl- ou monométhoxy-phényle, un radical difluoro, dichloro-, dibromo-phényle ou un radical méthyl-phényle substitué dans la portion aromatique par un atome de fluor, de chlore ou de brome et $X_1$, $X_2$ et $X_3$, qui sont identiques ou différents, représentent chacun hydrogène, chlore, brome ou méthoxy, à condition que lorsqu'ils sont identiques, $X_1$, $X_2$ et $X_3$ ne sont pas simultanément hydrogène.

4. Ethyl-2 (dibromo-3,5 hydroxy-4 benzoyl)-3 indolizine.

5. Bromo-1 n-propyl-2 (dibromo-3,5 hydroxy-4 benzoyl)-3 indolizine.

6. Isopropyl-2 (dibromo-3,5 hydroxy-4 benzoyl)-3 indolizine.

7. Bromo-1 phényl-2 (bromo-3 hydroxy-4 benzoyl)-3 indolizine.

8. Bromo-1 (fluoro-4 phényl)-2 (bromo-3 hydroxy-4 benzoyl)-3 indolizine.

9. Méthoxy-1 phényl-2 (bromo-3 hydroxy-4 benzoyl)-3 indolizine.

10. (Bromo-4 phényl)-2 (bromo-3 hydroxy-4 benzoyl)-3 indolizine.

11. Procédé de préparation de dérivés d'indolizine correspondant à la formule générale:

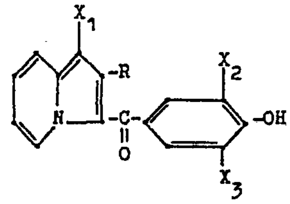

dans laquelle R représente un radical alkyle, linéaire ou ramifié, ayant de 1 à 8 atomes de carbone, un groupement phényle ou un groupement phényle portant un ou deux substituants, identiques ou différents choisis parmi des atomes d'halogène et parmi des groupements alkyles inférieurs et alkoxy inférieurs et $X_1$, $X_2$ et $X_3$, qui sont identiques ou différents, représentent chacun hydrogène, chlore, brome ou méthoxy, à condition que lorsqu'ils sont identiques $X_1$, $X_2$ et $X_3$ ne sont pas simultanément hydrogène, caractérisé en ce que:

(A) un dérivé d'indolizine de formule générale:

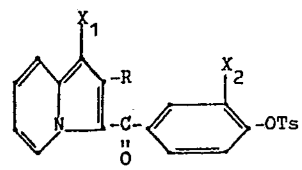

dans laquelle $X_1$ représente hydrogène ou méthoxy, $X_2$ représente chlore, brome ou méthoxy, Ts représente un radical p-toluènesulfonyle et R a la même signification que précédemment, est chauffé au reflux dans un solvant approprié et avec un hydroxyde de métal alcalin pour donner le sel métallique correspondant qui est hydrolisé, par la suite, à température ambiante en présence d'un acide hydrohalique pour obtenir le composé désiré dans lequel $X_1$ représente hydrogène ou méthoxy, $X_2$ représente chlore, brome ou méthoxy et $X_2$ est hydrogène.

(B) une alkyl-2 ou aryl-2 indolizine de formule générale:

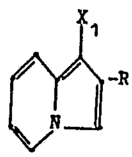

dans laquelle $X_1$ représente hydrogène ou méthoxy et R a la même signification que précédemment, est condensée à température ambiante et dans un solvant approprié, avec un chlorure d'acide p-hydroxybenzoïque de formule générale:

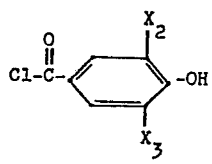

dans laquelle $X_2$ et $X_3$, qui sont identiques ou différents, représentent chlore, brome ou méthoxy, pour obtenir le composé désiré dans lequel $X_1$ représente hydrogène ou méthoxy et $X_2$ et $X_3$, qui sont les mêmes ou différents, représentent chacun chlore, brome ou méthoxy.

(C) un dérivé de benzoyl-indolizine de formule générale:

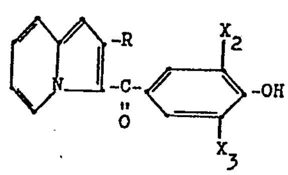

dans laquelle R a la même signification que précédemment et $X_2$ et $X_3$, qui sont identiques ou différents, représentent chacun hydrogène, chlore, brome ou méthoxy est mis en réaction avec du brome à la température ambiante dans un solvant approprié et en présence d'un acétate de métal alcalin, pour obtenir le composé désiré dans lequel $X_1$ représente brome et $X_2$ et $X_3$, qui sont identiques ou différents, représentent chacun hydrogène, chlore, brome ou méthoxy.

(D) un dérivé de benzoyl-indolizine de formule générale:

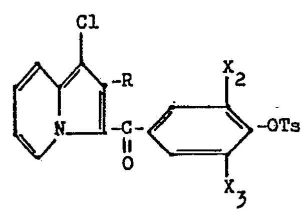

dans laquelle R et Ts ont la même signification que précédemment et $X_2$ et $X_3$, qui sont identiques ou différents, représentent chacun hydrogène, chlore, brome ou méthoxy, est chauffé au reflux, dans un solvant approprié, avec un hydroxide de métal alcalin pour obtenir le sel de métal alcalin

correspondant qui est ensuite hydrolysé en présence d'un acide hydrohalique pour donner le dérivé désiré d'indolizine dans lequel $X_1$ représente chlore et $X_2$ et $X_3$, qui sont identiques ou différents, représentent chacun hydrogène, chlore, brome ou méthoxy.

12. Procédé selon la Revendication 11 caractérisé en ce que les atomes d'halogène sont des atomes de fluor, de chlore ou de brome et les groupements alkyles inférieurs et alkoxy inférieurs sont les groupements méthyle et méthoxy.

13. Procédé selon la Revendication:

a) 11 A ou 11 D dans laquelle l'hydroxyde de métal alcalin est l'hydroxyde de sodium

b) 11 A ou 11 D dans laquelle l'acide hydrohalique est l'acide chlorhydrique

c) 11 A ou 11 B ou 11 D dans laquelle le solvant est le dichloréthane

d) 11 C dans laquelle le solvant est le dioxane ou l'acide acétique et l'acétate de métal alcalin est l'acétate de sodium.

14. Composition pharmaceutique ou vétérinaire contenant, comme principe actif, au moins un dérivé d'indolizine selon la Revendication 1 ou 2, en association avec un véhicule pharmaceutique ou un excipient approprié.

15. Composition pharmaceutique ou vétérinaire contenant comme principe actif au moins un dérivé d'indolizine selon l'une quelconque des Revendications 4 à 10.

**Patentansprüche**

1. Indolizinderivate der allgemeinen Formel

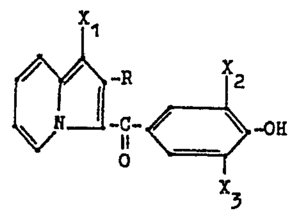

in welcher R für einen linearen oder verzweigten Alkylrest mit 1 bis 8 Kohlenstoffatomen, eine Phenylgruppe oder eine Phenylgruppe mit ein oder zwei identischen oder unterschiedlichen Substituenten, ausgewählt aus Halogenatomen und niedrigen Alkyl- und niedrigen Alkoxygruppen, steht und $X_1$, $X_2$ und $X_3$, die gleich oder verschieden sind, jeweils für Wasserstoff, Chlor, Brom oder Methoxy stehen, mit der Bedingung, daß — wenn $X_1$, $X_2$ und $X_3$ identisch sind — sie nicht alle gleichzeitig für Wasserstoff stehen.

2. Indolizinderivate nach Anspruch 1, dadurch gekennzeichnet, daß die Halogenatome Fluor, Chlor oder Brom und die niedrigen Alkyl- und niedrigen Alkoxygruppen Methyl- bzw. Methoxygruppen sind.

3. Indolizinderivate nach Anspruch 1, dadurch gekennzeichnet, daß R für einen linearen oder verzweigten Alkylrest mit 1 bis 8 Kohlenstoffatomen, einen Phenylrest, einen Monofluor-, Monochlor-, Monobrom-, Monomethyl- oder Monomethoxyphenylrest, einen Difluor-, Dichlor- oder Dibromphenylrest oder einen Methylphenylrest steht, der im aromatischen Teil durch ein Fluor-, Chlor- oder Bromatom substituiert ist, und $X_1$, $X_2$ und $X_3$, die gleich oder verschieden sind, jeweils für Wasserstoff, Chlor, Brom oder Methoxy stehen, mit der Bedingung, daß — wenn sie identisch sind — $X_1$, $X_2$ und $X_3$ nicht gleichzeitig Wasserstoff bedeuten.

4. 2-Äthyl-3-(3,5-Dibrom-4-hydroxybenzoyl)-indolizin.

5. 1-Brom-2-n-propyl-3-(3,5-dibrom-4-hydroxybenzoyl)-indolizin.

6. 2-Isopropyl-3-(3,5-dibrom-4-hydroxybenzoyl)-indolizin.

7. 1-Brom-2-phenyl-3-(3-brom-4-hydroxybenzoyl)-indolizin.

8. 1-Brom-2-(4-fluorphenyl)-3-(3-brom-4-hydroxybenzoyl)-indolizin.

9. 1-Methoxy-2-phenyl-3-(3-brom-4-hydroxybenzoyl)-indolizin.

10. 2-(4-Bromphenyl)-3-(3-brom-4-hydroxybenzoyl)-indolizin.

11. Verfahren zur Herstellung von Indolizinderivaten der allgemeinen Formel

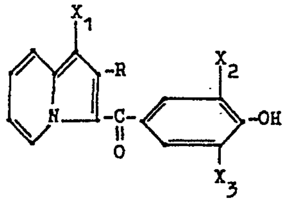

in welcher R für einen linearen oder verzweigten Alkylrest mit 1 bis 8 Kohlenstoffatomen, eine Phenylgruppe oder eine Phenylgruppe mit ein oder zwei identischen oder unterschiedlichen Substituenten,

ausgewählt aus Halogenatomen und niedrigen Alkyl- und niedrigen Alkoxygruppen, steht und $X_1$, $X_2$ und $X_3$, die gleich oder verschieden sind, jeweils für Wasserstoff, Chlor, Brom oder Methoxy stehen, mit der Bedingung, daß — wenn $X_1$, $X_2$ und $X_3$ identisch sind — sie nicht alle gleichzeitig für Wasserstoff stehen, dadurch gekennzeichnet, daß man

(A) ein Indolizinderivat der allgemeinen Formel:

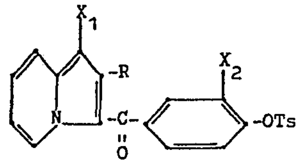

in welcher $X_1$ für Wasserstoff oder Methoxy steht, $X_2$ Chlor, Brom oder Methoxy bedeutet, Ts für einen p-Toluolsulfonylrest steht und R die oben angegebene Bedeutung hat, in einem geeigneten Lösungsmittel und mit einem Alkalimetallhydroxid zum Rückfluß erhitzt, um das entsprechende Metallsalz zu liefern, das anschließend bei Zimmertemperatur in Anwesenheit einer Halogenwasserstoffsäure hydrolysiert wird, um die gewünschte Verbindung zu erhalten, in welcher $X_1$ für Wasserstoff oder Methoxy steht, $X_2$ Chlor, Brom oder Methoxy bedeutet und $X_3$ für Wasserstoff steht;

(B) ein 2-Alkyl- oder 2-Arylindolizin der allgemeinen Formel

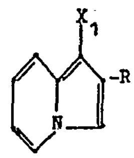

in welcher $X_1$ für Wasserstoff oder Methoxy steht und R die oben angegebene Bedeutung hat, bei Zimmertemperatur und in einem geeigneten Lösungsmittel mit einem Säurechlorid von p-Hydroxybenzoesäuren der allgemeinen Formel

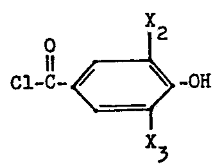

in welcher $X_2$ und $X_3$, die gleich oder verschieden sind, für Chlor, Brom oder Methoxy stehen, zur gewünschten Verbindung kondensiert, in welcher $X_1$ für Wasserstoff oder Methoxy steht und $X_2$ und $X_3$, die gleich oder verschieden sind, jeweils für Chlor, Brom oder Methoxy stehen;

(C) ein Benzoylindolizinderivat der allgemeinen Formel

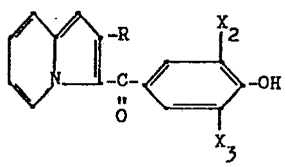

in welcher R die oben angegebene Bedeutung hat und $X_2$ und $X_3$, die gleich oder verschieden sind, jeweils für Wasserstoff, Chlor, Brom oder Methoxy stehen, mit Brom bei Zimmertemperatur in einem geeigneten Lösungsmittel und in Anwesenheit eines Alkalimetallacetates zur gewünschten Verbindung umsetzt, in welcher $X_1$ für Brom steht und $X_2$ und $X_3$, die gleich oder verschieden sind, jeweils für Wasserstoff, Chlor, Brom oder Methoxy stehen;

(D) ein Benzoylindolizinderivat der allgemeinen Formel

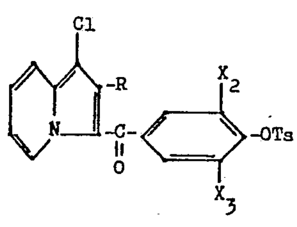

in welcher R und Ts die oben angegebene Bedeutung haben und $X_2$ und $X_3$, die gleich oder verschieden sind, jeweils für Wasserstoff, Chlor, Brom oder Methoxy stehen, in einem geeigneten Lösungsmittel mit einem Alkalimetallhydroxid zum Rückfluß erhitzt, um das entsprechende Alkalimetallsalz zu erhalten, das dann in Anwesenheit einer Halogenwasserstoffsäure zum gewünschten Indolizinderivat hydrolysiert wird, in welchem $X_1$ für Chlor steht und $X_2$ und $X_3$, die gleich oder verschieden sind, jeweils Wasserstoff, Chlor, Brom oder Methoxy bedeuten.

12. Verfahren nach Anspruch 11, dadurch gekennzeichnet, daß die Halogenatome solche von Fluor, Chlor oder Brom und die niedrigen Alkyl- und niedrigen Alkoxygruppen Methyl- bzw. Methoxygruppen sind.

13. Verfahren nach Anspruch:

a) 11 (A) oder 11 (D), in welchem das Alkalimetallhydroxid Natriumhydroxid ist

b) 11 (A) oder 11 (D), in welchem die Halogenwasserstoffsäure Salzsäure ist

c) 11 (A) oder 11 (B) oder 11 (D), in welchem das Lösungsmittel Dichloräthan ist

d) 11 (C), in welchem das Lösungsmittel Dioxan oder Essigsäure und das Alkalimetallacetat Natriumacetat ist.

14. Pharmazeutisches oder veterinärmedizinisches Präparat, enthaltend als aktiven Bestandteil mindestens ein Indolizinderivat gemäß Anspruch 1 oder 2 in Verbindung mit einem pharmazeutischen Träger oder einem geeigneten Streckmittel.

15. Pharmazeutisches oder veterinärmedizinisches Präparat, enthaltend als aktiven Bestandteil mindestens ein Indolizinderivat nach einem der Ansprüche 4 bis 10.

## Claims

1. Indolizine derivatives corresponding to the general formula:

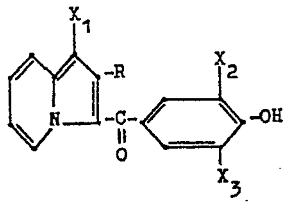

wherein R represents a straight- or branched-chain alkyl radical having from 1 to 8 carbon atoms, or a phenyl group non-substituted or bearing one or two substituents, which may be the same or different, selected from halogen atoms and from lower alkyl and alkoxy groups and $X_1$, $X_2$ and $X_3$, which may be the same or different, each represent hydrogen, chlorine, bromine or methoxy with the proviso that when they are identical $X_1$, $X_2$ and $X_3$ are not simultaneously hydrogen.

2. Indolizine derivatives according to Claim 1 wherein the halogen atoms are fluorine, chlorine or bromine atoms and the lower alkyl and alkoxy groups are methyl and methoxy groups.

3. Indolizine derivatives according to Claim 1 wherein R represents a straight- or branched-chain alkyl radical having from 1 to 8 carbon atoms, a phenyl radical, a mono-fluoro-, mono-chloro-, monobromo-, mono-methyl- or mono-methoxy-phenyl radical, a di-fluoro, di-chloro-, di-bromo-phenyl radical or a methyl-phenyl radical substituted in the aromatic moiety by an atom of fluorine, chlorine or bromine and $X_1$, $X_2$ and $X_3$, which may be the same or different, each represent hydrogen, chlorine, bromine or methoxy with the proviso that when they are identical, $X_1$, $X_2$ and $X_3$ are not simultaneously hydrogen.

4. 2-Ethyl-3-(3,5-dibromo-4-hydroxy-benzoyl)-indolizine.

5. 1-Bromo-2-n-propyl-3-(3,5-dibromo-4-hydroxy-benzoyl)-indolizine.

6. 2-Isopropyl-3-(3,5-dibromo-4-hydroxy-benzoyl)-indolizine.

7. 1-Bromo-2-phenyl-3-(3-bromo-4-hydroxy-benzoyl)-indolizine.

8. 1-Bromo-2-(4-fluoro-phenyl)-3-(3-bromo-4-hydroxy-benzoyl)-indolizine.

9. 1-Methoxy-2-phenyl-3-(3-bromo-4-hydroxy-benzoyl)-indolizine.

10. 2-(4-Bromo-phenyl)-3-(3-bromo-4-hydroxy-benzoyl)-indolizine.

11. A process for preparing indolizine derivatives corresponding to the general formula:

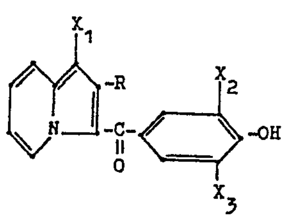

in which R represents a straight- or branched-chain alkyl radical having from 1 to 8 carbon atoms, or a phenyl group non-substituted or bearing one or two substituents, which may be the same or different, selected from halogen atoms and from lower alkyl and alkoxy groups, and $X_1$, $X_2$ and $X_3$, which may be the same or different, each represent hydrogen, chlorine, bromine or methoxy with the proviso that when they are identical $X_1$, $X_2$ and $X_3$ are not simultaneously hydrogen, whereby:

A) an indolizine derivative of the general formula:

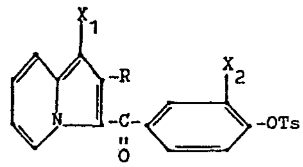

in which $X_1$ represents hydrogen or methoxy, $X_2$ represents chlorine, bromine or methoxy, Ts represents a p-toluenesulphonyl radical and R has the same meaning as given above, is refluxed in a suitable solvent with an alkali metal hydroxide so as to provide the corresponding metal salt which is subsequently hydrolysed at room-temperature in the presence of a hydrohalic acid to obtain the required compound in which $X_1$ represents hydrogen or methoxy, $X_2$ represents chlorine, bromine or methoxy and $X_3$ is hydrogen.

B) a 2-alkyl- or 2-aryl-iodolizine of the general formula:

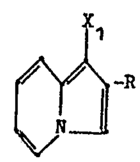

in which $Z_1$ represents hydrogen or methoxy and R has the same meaning as given above, is condensed at room-temperature in a suitable solvent with a p-hydroxybenzoic acid chloride derivative of the general formula:

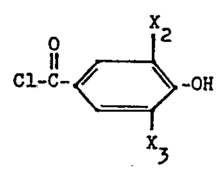

in which $X_2$ and $X_3$, which are the same or different, represent chlorine, bromine or methoxy, to obtain the required compound in which $X_1$ represents hydrogen or methoxy and $X_2$ and $X_3$, which are the same or different, each represent chlorine, bromine or methoxy.

C) a benzoyl-indolizine derivative of the general formula:

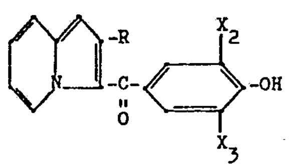

in which R has the same meaning as given above and $X_2$ and $X_3$, which are the same or different, each represent hydrogen, chlorine, bromine or methoxy, is reacted with bromine at room-temperature, in a suitable solvent and in the presence of an alkali metal acetate, to obtain the required compound in which $X_1$ represents bromine and $X_2$ and $X_3$, which are the same or different, each represent hydrogen, chlorine, bromine or methoxy.

D) a benzoyl-indolizine derivative of the general formula:

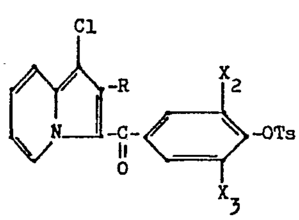

in which R and Ts have the same meaning as given above and $X_2$ and $X_3$, which are the same or different, each represent hydrogen, chlorine, bromine or methoxy, is refluxed in a suitable solvent with an alkali metal hydroxide so as to provide the corresponding alkali metal salt which is subsequently hydrolysed in the presence of a hydrohalic acid to obtain the required indolizine derivative in which $X_1$ represents chlorine, $X_2$ and $X_3$, which are the same or different, each represent hydrogen, chlorine, bromine or methoxy.

12. Process according to Claim 11 whereby the halogen atoms are fluorine, chlorine or bromine atoms and the lower alkyl and alkoxy groups are methyl and methoxy groups.

13. Process according to claim:

a) 11 A or 11 D in which the alkali metal hydroxide is sodium hydroxide

b) 11 A or 11 D in which the hydrohalic acid is hydrochloric acid

c) 11 A or 11 B or 11 D in which the solvent is dichlorethane

d) 11 C in which the solvent is dioxan or acetic acid and the alkali metal acetate is sodium acetate.

14. A pharmaceutical or veterinary composition containing, as active principle, at least one indolizine derivative according to Claim 1 or 2, in association with a pharmaceutical carrier or excipient therefor.

15. A pharmaceutical or veterinary composition containing as active ingredient at least one indolizine derivative according to Claims 4 to 10.

FIG. 1

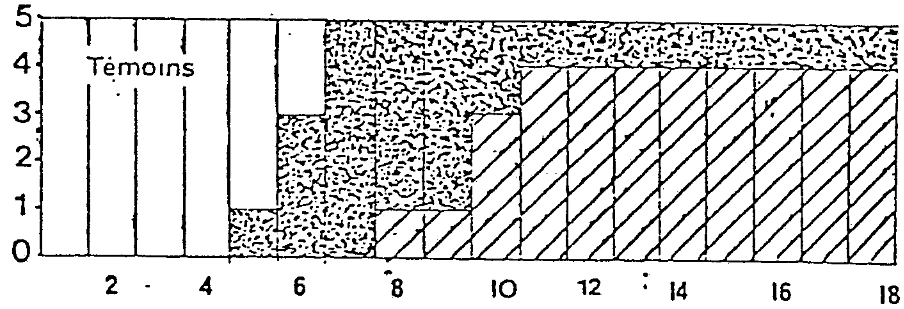


FIG. 2

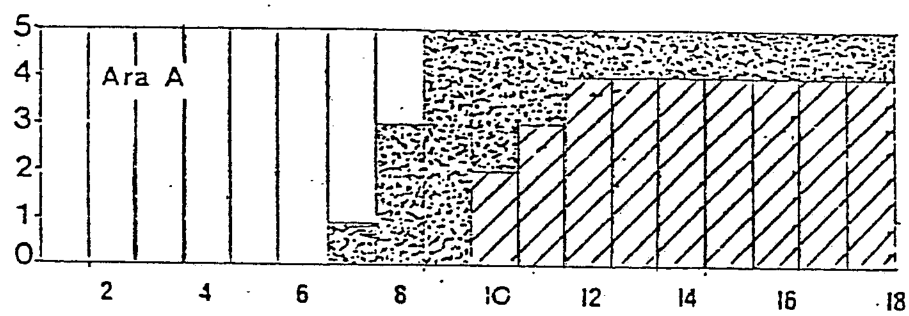


FIG. 3

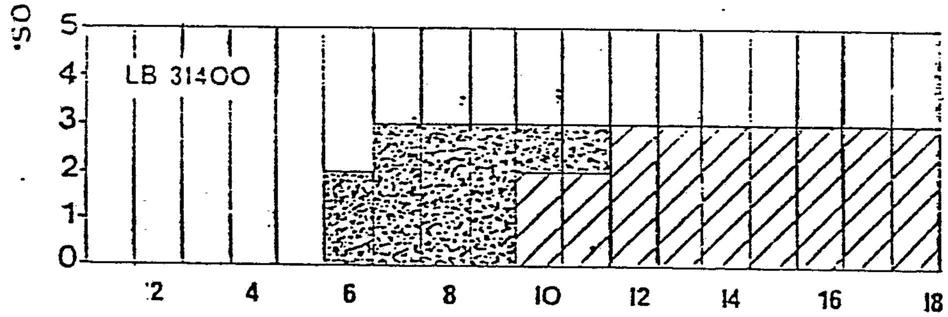


FIG. 4